(19) Europäisches Patentamt
European Patent Office
Office européen des brevets

(11) Numéro de publication: **0 065 907**
**B1**

(12) # FASCICULE DE BREVET EUROPEEN

(45) Date de publication du fascicule du brevet: **05.12.84**

(51) Int. Cl.³: **C 07 D 401/06**, A 61 K 31/44

(21) Numéro de dépôt: **82400871.8**

(22) Date de dépôt: **11.05.82**

(54) **((Tétra- et hexahydro pyridyl-4)-2 éthyl)-3 indoles et leur utilisation comme médicaments.**

(30) Priorité: **22.05.81 FR 8110218**

(43) Date de publication de la demande: **01.12.82 Bulletin 82/48**

(45) Mention de la délivrance du brevet: **05.12.84 Bulletin 84/49**

(84) Etats contractants désignés: **BE CH DE FR GB IT LI LU NL SE**

(56) Documents cités:
**EP - A - 0 007 258**
**FR - M - 6 291**
**GB - A - 925 429**
**GB - A - 1 023 781**

**THE JOURNAL OF ORGANIC CHEMISTRY, vol. 26, no. 7, septembre 1961, A.P. GRAY et al.: "The catalytic hydrogenation of indolylethylpyridines. 4-(Indolylethyl)-1-aralkylpiperidines as potent analgesics, pages 3368-3373**

(73) Titulaire: **PHARMUKA LABORATOIRES, 35 Quai du Moulin de Cage, F-92231 Gennevilliers (FR)**

(72) Inventeur: **Audiau, François, 9, rue Guérin, F-94220 Charenton (FR)**
Inventeur: **Le Fur, Gérard Roger, 35, rue du Progrès, F-92350 Plessis-Robinson (FR)**

(74) Mandataire: **Gaumont, Robert et al, RHONE-POULENC RECHERCHES Service Brevets Pharma 25, Quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

Le brevet français N° 75.38051 (N° 2334358) déposé le 12 décembre 1975 décrit des dérivés de l'indole, actifs comme inhibiteurs spécifiques de la recapture de la sérotonine par les neurones et pouvant de ce fait trouver des applications comme médicaments psychotropes, plus particulièrement comme antidépresseurs. Comme tels on peut mentionner spécialement le [(pipéridyl-4)-2 éthyl]-3 indole de formule:

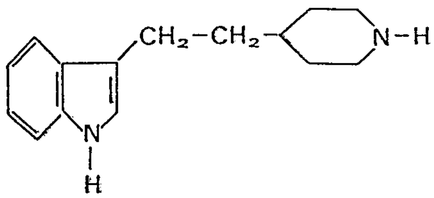

(I)

ou indalpine G. Le Fur et coll., Life Sciences, *23*, 1959 (1978).

La présente invention a pour objet, à titre de produits nouveaux, les composés de formule générale:

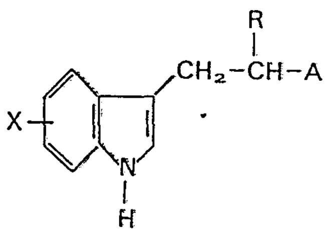

(II)

dans laquelle soit R représente un atome d'hydrogène ou un groupe alkyl possédant 1 à 3 atomes de carbone, X représente un atome d'hydrogène ou d'halogène et A représente le radical tétrahydro-1,2,5,6 pyridyl-4, soit R représente un groupe alkyle possédant 1 à 3 atomes de carbone, X représente un atome d'hydrogène ou d'halogène et A représente le radical hexahydro-1,2,3,4,5,6 pyridyl-4.

Le radical hexahydro-1,2,3,4,5,6 pyridyl-4 s'entend aussi, en nomenclature traditionnelle, pour le radical pipéridyl-4.

Il a été trouvé, conformément à la présente invention faite dans les services de la demanderesse, que les composés de formule (II), telle que définie ci-dessus, sont capables non seulement, comme l'indalpine, d'inhiber la recapture de la sérotonine mais aussi de provoquer la libération de la sérotonine contenue soit dans les neurones, soit dans les plaquettes sanguines.

Cette double fonction se manifeste par une action plus intense et plus rapide sur la dépression en raison de la libération neuronale de sérotonine dans la fente synaptique, conjuguée à l'inhibition de la recapture. La libération dans le plasma sanguin de la sérotonine plaquettaire améliore les états migraineux; enfin, la déplétion en sérotonine des plaquettes prévient la formation de thrombi artériels.

Les composés selon l'invention peuvent être préparés par réduction des [(pyridyl-4)-2 éthyl]-3 indoles correspondants de formule générale:

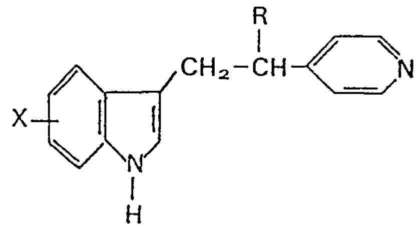

(III)

Cette formule ne comprend pas le [(pyridyl-4)-2 éthyl]-3 indole, composé connu utilisable pour la préparation de l'indalpine. Les composés de formule (III) utilisables pour préparer les nouveaux dérivés tétra- et hexahydro selon l'invention sont eux-mêmes des produits intermédiaires nouveaux. Leur réduction peut être conduite suivant le procédé de S. Wawzonek et coll. J. Am. Chem. Soc. vol. 74, p. 28, 1952.

Une méthode avantageuse consiste à effectuer la réduction au moyen de sodium au sein d'un alcool ayant 1 à 5 atomes de carbone tel que le n-butanol, à une température comprise entre 80 et 120°C. Dans ces conditions on obtient un mélange de produits totalement saturés (formule IV) et partiellement saturés (formule (V):

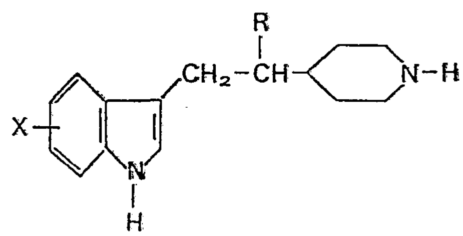

(IV)

dérivé hexahydropyridyle ou pipéridyle

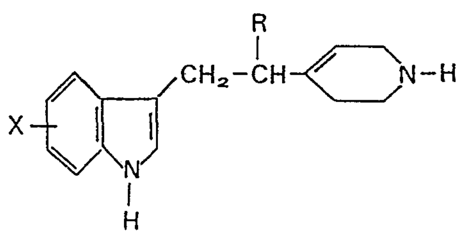

(V)

dérivé tétrahydro-1,2,5,6.

La séparation des produits de la réaction peut être effectuée par chromatographie et cristallisation des produits sous forme de base ou de sels.

Les produits de formule (IV) dans laquelle X représente l'atome d'hydrogène peuvent également être préparés par hydrogénation catalytique des composés de formule (III) correspondants ou de leurs sels. Cette hydrogénation est effectuée au sein d'un solvant inerte, à une température comprise entre 20 et 100°C et sous une pression d'hydrogène de 1 à 50 bars. Comme solvant on peut utiliser par exemple des alcools tels que le méthanol ou l'éthanol ou des acides tels que l'acide acétique. Comme catalyseur d'hydrogénation on peut utiliser le nickel, le palladium, le rhodium, le ruthénium ou le platine.

Les produits de départ de formule (III) peuvent être préparés par réaction d'un dérivé de l'indole de formule:

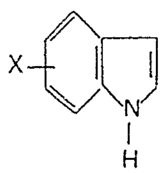

(VI)

avec un dérivé de la pyridine de formule:

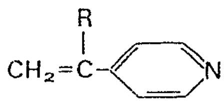

(VII)

X et R ayant les mêmes significations que dans la formule (III). On opère avantageusement au sein de l'acide acétique à une température comprise entre 100 et 120°C.

Les mélanges réactionnels obtenus suivant les divers procédés décrits précédemment sont traités suivant les méthodes classiques, physiques (évaporation, extraction à l'aide d'un solvant, distillation, cristallisation, chromatographie, etc.) ou chimiques (formation de sel et régénération de la base, etc.) afin d'isoler les composés de formule (II) à l'état pur.

Les composés de formule (II) sous forme de bases libres peuvent éventuellement être transformés en sels d'addition avec un acide minéral ou organique par action d'un tel acide au sein d'un solvant approprié.

Les exemples suivants illustrent l'invention sans la limiter.

*Exemple 1: [(Tétrahydro-1,2,5,6 pyridyl-4)-2 éthyl]-3 indole*

On chauffe sous azote vers 90°C une solution de 10,3 g de [(pyridyl-4)-2 éthyl]-3 indole (préparé selon le brevet US 3300506) dans 400 ml de n-butanol. On ajoute ensuite par portions 8 g de sodium tout en agitant magnétiquement, et continue le chauffage vers 110°C jusqu'à disparition du sodium. Après retour à la température ambiante on verse dans 400 ml d'eau, lave la phase organique à l'eau jusqu'à neutralité, sèche sur sulfate de sodium et évapore sous vide. On reprend le résidu d'évaporation avec 25 ml d'éthanol absolu et purifie par chromatographie sur gel de silice (éluant: éthanol-diéthylamine=40-1). Après deux recristallisations dans le méthanol on obtient 2,6 g de [(tétrahydro-1,2,5,6 pyridyl-4)-2 éthyl]-3 indole fondant à 155°C.

*Exemple 2: [(Tétrahydro-1,2,5,6 pyridyl-4)-2 méthyl-2 éthyl]-3 indole*

On opère comme à l'exemple 1 à partir de 11,2 g de [(pyridyl-4)-2 méthyl-2 éthyl]-3 indole; on obtient après purification par chromatographie sur gel de silice (éluant: éthanol-diéthylamine: 40-1) et cristallisation dans l'éthanol absolu 3,8 g de [(tétrahydro-1,2,5,6 pyridyl-4)-2 méthyl-2 éthyl]-3 indole fondant à 125°C.

Le produit de départ est préparé de la façon suivante: on porte à reflux pendant 24 h: 100 g d'indole, 450 ml d'acide acétique et 21,3 g d'α-méthylvinyl-4 pyridine (préparée selon Clemo et Hoggarth: J.C.S. 1941, 41-7). On verse ensuite dans 1 l d'eau glacée, alcalinise par de l'hydroxyde de sodium, extrait par 3×500 ml d'acétate d'éthyle et lave à l'eau la phase organique; on effectue sur celle-ci 3 extractions à l'acide chlorhydrique normal, lave à l'acétate d'éthyle la solution acide aqueuse, alcalinise celle-ci à l'ammoniaque et extrait l'huile qui relargue avec 2×500 ml d'acétate d'éthyle; on évapore le solvant sous pression réduite et purifie le résidu par chromatographie sur gel de silice (éluant: acétate d'éthyle). Après cristallisation dans un mélange de toluène et d'éther de pétrole (1-1), on obtient 11,2 g de [(pyridyl-4)-2 méthyl-2 éthyl]-3 indole fondant à 115°C.

*Exemple 3: [(Tétrahydro-1,2,5,6 pyridyl-4)-2 éthyl]-3 fluoro-5 indole*

On opère comme à l'exemple 1, à partir de 12 g de [(pyridyl-4)-2 éthyl]-3 fluoro-5 indole; on obtient après purification par chromatographie sur gel de silice (éluant: toluène-éthanol-diéthylamine:10-10-1) 3,5 g de [(tétrahydro-1,2,5,6 pyridyl-4)-2 éthyl]-3 fluoro-5 indole dont le fumarate acide fond à 208-210°C.

Le produit de départ est préparé suivant un procédé analogue à celui du dernier paragraphe de l'exemple 2: on porte à reflux pendant 6 h 9,5 g de fluoro-5 indole (brevet US 879619) et 11 g de vinyl-4 pyridine dans 50 ml d'acide acétique. On verse ensuite sur 300 g de glace pilée et alcalinise avec une solution de carbonate de potassium à 20%. Un précipité se forme, que l'on essore et lave plusieurs fois à l'eau. Après séchage sous vide on obtient 12 g de [(pyridyl-4)-2 éthyl]-3 fluoro-5 indole fondant à 128°C.

*Exemple 4: [(Pipéridyl-4)-2 méthyl-2 éthyl]-3 indole*

On opère comme à l'exemple 2: après élution du [(tétrahydro-1,2,5,6 pyridyl-4)-2 méthyl-2 éthyl]-3 indole, on poursuit la chromatographie avec le mélange éthanol-diéthylamine (9-1). Après cristallisation dans l'éthanol absolu, on obtient 2,3 g de [(pipéridyl-4)-2 méthyl-2 éthyl]-3 indole fondant à 150°C.

*Exemple 5:*

On hydrogène sous pression ordinaire, à 60°C pendant 15 h, 0,8 g de [(pyridyl-4)-2 méthyl-2 éthyl]-3 indole dans 20 ml d'acide acétique en présence de 0,4 g d'oxyde de platine. Le catalyseur est éliminé par filtration et le filtrat est évaporé. Le résidu est purifié par chromatographie sur silice (éluant: éthanol-diéthylamine: 9-1). On obtient 0,25 g de [(pipéridyl-4)-2 méthyl-2 éthyl]-3 indole qu'on transforme en chlorhydrate au sein de l'éthanol; on obtient 0,15 g de chlorhydrate de [(pipéridyl-4)-2 méthyl-2 éthyl]-3 indole fondant à 210°C.

*Propriétés pharmacologiques*

On sait que la recapture de la sérotonine par les plaquettes sanguines constitue un bon modèle de la recapture de cette amine par les neurones (cf. J. Tuomisto, J. Pharm., Pharmac., *26*, 92 (1974).

*Propriétés pharmacologiques*

On sait que la recapture de la sérotonine par les plaquettes sanguines constitue un bon modèle de la recapture de cette amine par les neurones [cf. J. Tuomisto, «J. Pharm., Pharmac.», *26*, 92 (1974)]. Appliquée à l'étude des médicaments, une méthode mettant en œuvre les plaquettes sanguines présente le grand intérêt de permettre l'utilisation de cellules humaines, ce qui lui confère un bon caractère prévisionnel.

La capacité des produits à inhiber la recapture de la sérotonine ou à provoquer sa libération a été mise en évidence sur des plaquettes sanguines humaines, selon J.L. David et coll., «Platelets Function and thrombosis, a review of methods», p. 335 (Plenum Press, London 1972).

1) *Inhibition de la recapture de la sérotonine*

Les résultats sont exprimés par une dose inhibitrice 50% ou $I_{50}$ qui représente la dose de produit, en micromoles par litre, diminuant de 50% la recapture de la sérotonine.

2) *Libération de la sérotonine*

L'action des produits sur la libération de la sérotonine est testée à la concentration de $5 \times 10^{-5}$ mol/l. Les résultats obtenus sont exprimés en pourcentage d'augmentation de la libération de la sérotonine par rapport aux résultats fournis par les témoins.

Les résultats obtenus avec les composés selon l'invention sont rassemblés dans le tableau ci-dessous. Dans ce tableau figurent, à titre comparatif, les résultats obtenus avec deux produits de référence (imipramine et p-chloroamphétamine) et avec l'indalpine.

| Produit | Inhibition de la recapture de la sérotonine $I_{50}$ (micromol/l) | Pourcentage d'augmentation de la libération de la sérotonine (concentration du produit: $5 \times 10^{-5}$ mol/l) |
|---|---|---|
| Exemple 1 | 0,055 | 85 |
| Exemple 2 | 0,04 | 68 |
| Exemple 4 | 0,01 | 76 |
| Indalpine | 0,035 | 22 |
| Imipramine | 0,4 | 13 |
| p-Chloroamphétamine | 12 | 51 |

Il ressort du tableau que les produits de l'invention sont non seulement de puissants inhibiteurs de recapture de la sérotonine (activités équivalentes à celle de l'indalpine), mais en outre de puissants agents de libération de la sérotonine, encore plus actifs que la p-chloroamphétamine.

L'intérêt de ces composés réside dans le fait qu'ils induisent comme la parachloroamphétamine la libération de sérotonine, sans pourtant présenter aucune des autres propriétés pharmacologiques caractéristiques des amphétaminiques (anorexie, hypermotilité).

*Propriétés toxicologiques*

La toxicité aiguë des produits a été déterminée chez la souris mâle $CD_1$ (Charles River) par voie orale. La $DL_{50}$ calculée, après 3 jours d'observation, par la méthode cumulative de J.J. Reed et coll. («Am. J. Hyg.», *27*, 493, 1938) est de 600 mg/kg pour les composés des exemples 1 et 2 et d'environ 400 mg/kg pour le composé de l'exemple 4.

Les composés selon l'invention sont atoxiques à 100 mg/kg et se comportent donc comme des substances relativement peu toxiques chez la souris.

*Application thérapeutique*

Les composés de l'invention et leurs sels pharmaceutiquement acceptables peuvent être utilisés en thérapeutique humaine sous forme de comprimés, capsules, gélules, suppositoires, solutions ingérables ou injectables, etc., comme régulateurs du tonus vasculaire sérotonino-dépendant, notamment pour le traitement des migraines, comme agents antithrombosants et comme médicaments thymoanaleptiques à action particulièrement rapide (à cause de leur action sur la libération de la sérotonine).

La posologie dépend des effets recherchés et de la voie d'administration uitlisée. Par exemple, par voie orale, elle peut être comprise entre 15 et 250 mg de substance active par jour, avec des doses unitaires allant de 5 à 50 mg.

## Revendications

1. Composés de formule générale:

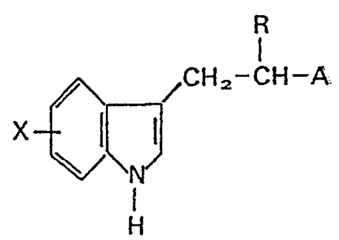

(II)

dans laquelle soit R représente un atome d'hydrogène ou un groupe alkyle possédant 1 à 3 atomes de carbone, X représente un atome d'hydrogène ou d'halogène et A représente le radical tétrahydro-1,2,5,6 pyridyl-4, soit R représente un groupe alkyle possédant 1 à 3 atomes de carbone, X représente un atome d'hydrogène ou d'halogène et A représente le radical hexahydro-1,2,3,4,5,6 pyridyl-4.

2. Procédé pour la préparation des composés revendiqués suivant la revendication 1, caractérisé en ce que les [(pyridyl-4)-2 éthyl]-3 indoles correspondants de formule générale:

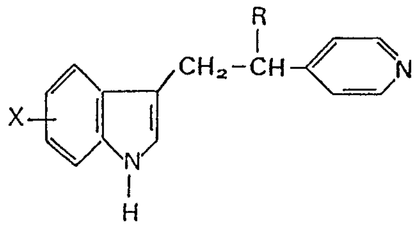

(III)

sont soumis à une réduction suivant un procédé connu en soi.

3. Procédé tel que défini suivant la revendication 2, caractérisé en ce que la réduction des composés de formule (III) est effectuée au moyen de sodium au sein d'un alcool possédant 1 à 5 atomes de carbone, à une température comprise entre 80 et 120°C.

4. Procédé tel que défini suivant la revendication 2, caractérisé en ce que la réduction des composés de formule (III), dans laquelle X représente l'hydrogène, est effectuée par hydrogénation catalytique.

5. En tant qu'agents inhibiteurs de recapture de la sérotonine et agents de libération de la sérotonine, utilisables comme médicaments, spécialement comme thymoanaleptiques antimigraineux et antithrombosants, les composés revendiqués selon la revendication 1 et leurs sels avec les acides pharmaceutiquement acceptables.

## Patentansprüche

1. Verbindungen der allgemeinen Formel (II):

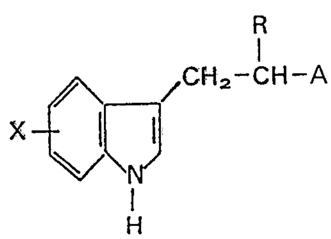

(II)

worin entweder R ein Wasserstoffatom oder eine Alkylgruppe mit 1 bis 3 Kohlenstoffatomen, X ein Wasserstoff- oder Halogenatom und A den Rest 1,2,5,6-Tetrahydro-4-pyridyl bedeuten; oder R eine Alkylgruppe mit 1 bis 3 Kohlenstoffatomen, X ein Wasserstoff- oder Halogenatom und A den Rest 1,2,3,4,5,6-Hexahydro-4-pyridyl bedeuten.

2. Verfahren zur Herstellung der Verbindungen gemäss Anspruch 1, dadurch gekennzeichnet, dass die 3-[2-(4-Pyridyl)äthyl]indole entsprechend der allgemeinen Formel (III):

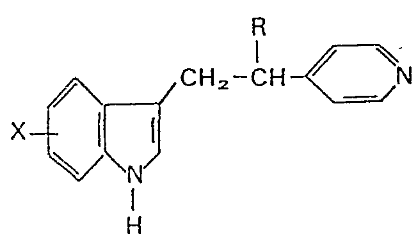

(III)

einer Reduktion nach einem an sich bekannten Verfahren unterworfen werden.

3. Verfahren gemäss Anspruch 2, daduch gekennzeichnet, dass die Reduktion der Verbindungen der Formel (III) mittels Natrium in einem Alkohol mit 1 bis 5 Kohlenstoffatomen bei einer Temperatur zwischen 80 und 120°C durchgeführt wird.

4. Verfahren gemäss Anspruch 2, dadurch gekennzeichnet, dass die Reduktion der Verbindungen der Formel (III), worin X Wasserstoff bedeutet, durch katalytische Hydrierung durchgeführt wird.

5. Als Inhibitoren der Wiederbindung des Serotonins und Mittel zur Freisetzung des Serotonins, die als Arzneimittel, besonders als Thymoanaleptika mit Antimigräne- und Antithrombosewirkung, anwendbar sind, die gemäss Anspruch 1 beanspruchten Verbindungen und ihre Salze mit pharmazeutisch annehmbaren Säuren.

## Claims

1. Compounds of the general Formula (II):

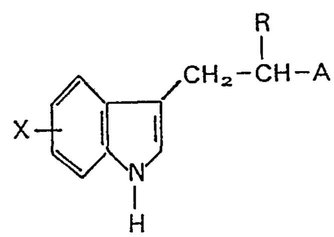

(II)

in which either R represents a hydrogen atom or an alkyl group possessing 1 to 3 carbon atoms, X represents a hydrogen atom or halogen atom and A represents the 1,2,5,6-tetrahydro-4-pyridyl radical, or R represents an alkyl group possessing 1 to 3 carbon atoms, X represents a hydrogen or halogen atom and A represents the 1,2,3,4,5,6-hexahydro-4-pyridyl radical.

2. Process for the preparation of the compounds according to Claim 1, characterised in that the corresponding 3-[2-(4-pyridyl)ethyl]indoles of the general Formula (III):

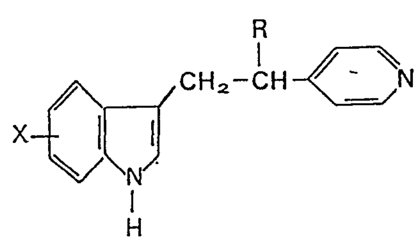

(III)

are subjected to a reduction according to a process known *per se*.

3. Process according to Claim 2, characterised in that the reduction of the compounds of the Formula (III) is carried out by means of sodium in an alcohol possessing 1 to 5 carbon atoms at a temperature of between 80 and 120°C.

4. Process according to Claim 2, characterised in that the reduction of the compounds of the Formula (III) in which X represents hydrogen is carried out by catalytic hydrogenation.

5. As inhibitors of the recapture of serotonine and liberators of serotonine, which may be used as medicaments, especially as thymo-analeptic, antimigraine and anti-thrombosis agents, the compounds claimed according to Claim 1 and their salts with pharmaceutically acceptable acids.